# EUROPEAN PATENT SPECIFICATION

(11) **EP 2 422 720 B1**
(45) Date of publication and mention of the grant of the patent: **24.04.2013**
(21) Application number: 11178004.5
(22) Date of filing: 18.08.2011
(51) Int. Cl.: A61B 17/22, A61B 17/28

(54) **Forceps**
Zange
Forceps

(30) Priority: 27.08.2010 TR 201007193
(43) Date of publication of application: 29.02.2012
(73) Proprietor: Adnan Menderes Universitesi, 09000 Aydin (TR)
(72) Inventor: Boylu, Sukru, 09000 AYDIN (TR); Bozdag, Ali Dogan, 09000 AYDIN (TR)
(74) Representative: Dericioglu, Ekin

(56) References cited:
- DE-A1- 2 945 407
- RU-C1- 2 340 297
- US-A- 4 509 517
- US-A- 5 693 069

## Description

### Field of the Invention

The present invention relates to forceps which are used in laparoscopic gallbladder surgery and enable the gallstones to be removed.

### Background of the Invention

In the state of the art, long, thin instruments are used in laparoscopic surgery. The said instruments are inserted to the abdomen during surgery and enable the necessary operation to be performed.

Laparoscopic instruments are inserted to the abdomen by passing through the hollow pipe-shaped instruments called trochar. At the end of the operation, the gallbladder is held with one of the laparoscopic instruments and it is tried to be removed out of the abdomen by being pulled with a trochar. When there are lots of gallstones and/or they are big, the gallbladder cannot be pulled out of the abdomen from the puncture done by the trochar. In this case the puncture of the trochar may need to be enlarged. As another solution, the part of the gallbladder pulled out of the abdomen can be punctured and the gallstones can be crushed into small pieces by inserting an instrument such as forceps through this puncture and crushing the stones. The crushed gallstones are crushed between the heads located at the ends of the forceps and the stones need to be evacuated by the forceps being removed. The said process needs to be repeated until almost all of the stones in the gallbladder have been removed .

United States patent document no US5693069, an application known in the state of the art, discloses forceps for crushing the stones in the gallbladder, but it is required to remove the forceps out of the gallbladder many times and clean the tips of the forceps and then insert into the gallbladder again during operation. This situation causes both time loss and it is risky. The gallbladder can be punctured during these operations; the gall may spill into the abdomen, stones and stone pieces may fall into the abdomen.

Russian Patent document no RU2340297, an application known in the state of the art, discloses a device which is inserted into the gallbladder and grabs, crushes and aspires the calculi. The said device enables the calculi therein to be seen and captured with the help of an endoscope which can pass through it by being inserted inside the gallbladder, the big calculus to be crushed and the small calculus to be aspired The said device is designed to extract the calculus by laying open the gallbladder before removing and extracting the gallbladder from its place. Laying open the gallbladder and extracting the calculus is not a factor that makes the operation easy before performing cholecystectomy except very rare conditions. Also there is no mechanism that will prevent the gall which will spill when the gallbladder is punctured; it may be risky that the gall disperses.

Chinese patent document no CN201127637, an application known in the state of the art, discloses a device which catches the gallstone with a basket. With the said method it is possible to catch the stones in the gallbladder; a couple of stones can be caught at the same time. Since the device is not strong enough to crush the stones, pulling the stones out of the puncture is difficult, the stones are needed to be caught and pulled one by one. In this case it is required to enter the gallbladder each time and there is a risk that the gallbladder may be damaged and/or punctured.

German patent document no DE4212430, an application known in the state of the art, discloses a device for chopping up the gallbladder and taking the pieces into a bag and extracting out of the abdomen. The histopathological examination of the gallbladder is very difficult and the staging of the patient cannot be performed in case of gallbladder malignancy. Also the mechanism of the device is not enough to crush large stones.

United States patent document no US5320627, an application known in the state of the art, discloses a cannula which has changeable tips in order to crush the stones and which can aspire. The tips of the said invention are not strong enough to crush the stones. Also the pointed ends can puncture the gallbladder. Also the long length of the device may cause uncontrolled entry into the gallbladder and the gallbladder may be punctured at this time.

United States patent document no US5215521, an application known in the state of the art, discloses a bag entering through the trochar and a device which aspirates the organ extracted into the bag by chopping. With the said device it is possible to chop an organ such as spleen and kidneys as it is soft tissue. But rigid formations such as calculi cannot be crushed.

United States patent document no US2004225192, an application known in the state of the art, discloses a surgical dilator extractor which is introduced into the abdominal cavity through the trochar cannula, forms a tissue receiving space at the distal end. The cone shaped part at the end of the device is made of a material having low friction such as polytetrafluoroethylene, and the inner surface has high friction such as sand crusher or rough surface. The invention is a device having a crusher end which is used to extract the calculi from the gallbladder in laparoscopic surgery. In the invention the calculus are extracted by being held by a grabber.

United States patent document no US4243040, an application known in the state of the art, discloses a device which is for extracting kidney stones, gall stones and the like from the human body, and comprises a thin tubular rubber sleeve connected with the distal end. The rubber sleeve enables the stone to be captured between the two nested tubes by being inflated by the fluid pressure, the captured stones are pulled inside and removed with the tubes. The invention is device that the stones inside the body are extracted from the body by means of a system comprised of sleeves. There is no crusher end present in the invention, capturing of the stone in the invention is enabled by the rubber structure which is inflated by the fluid pressure.

DE 2945407 is considered to represent the closest prior art and discloses forceps for kidney stone extraction, with tubular shafts for aspiration. No sliding connector is present.

### Summary of the Invention

The objective of the present invention is to realize forceps which enable the stones in the gallbladder to be crashed in order the gallbladder to remove from the trochar hole made on the abdomen wall after the laparoscopic gallbladder surgery.

Another objective of the present invention is to realize forceps which crash the stones by being inserted into the gallbladder once.

A further objective of the present invention is to realize forceps which extract the crushed stones by aspiration.

These objectives are achieved by the forceps as in appended claim 1.

### Detailed Description of the Invention

Forceps realized to fulfill the objectives of the present invention is illustrated in the accompanying figures, in which:

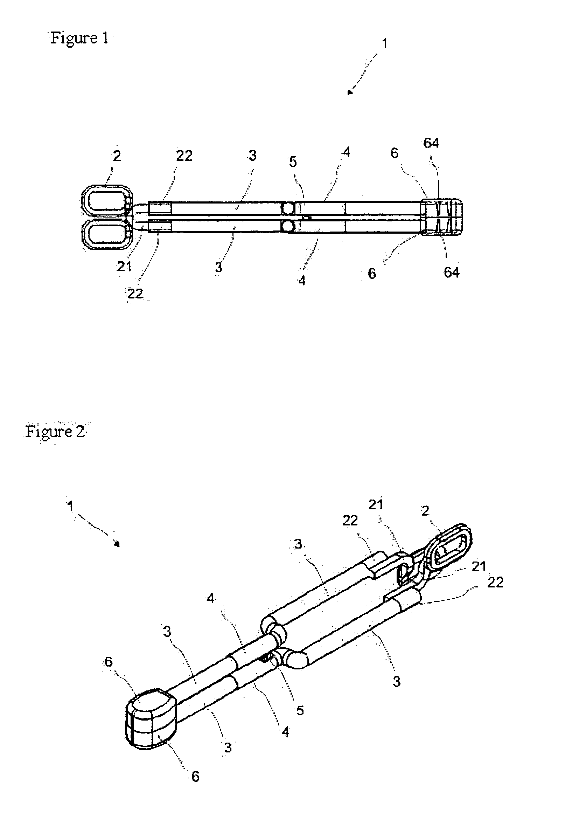

Figure 1 is the side view of the inventive forceps.
Figure -2 is the perspective view of the forceps.

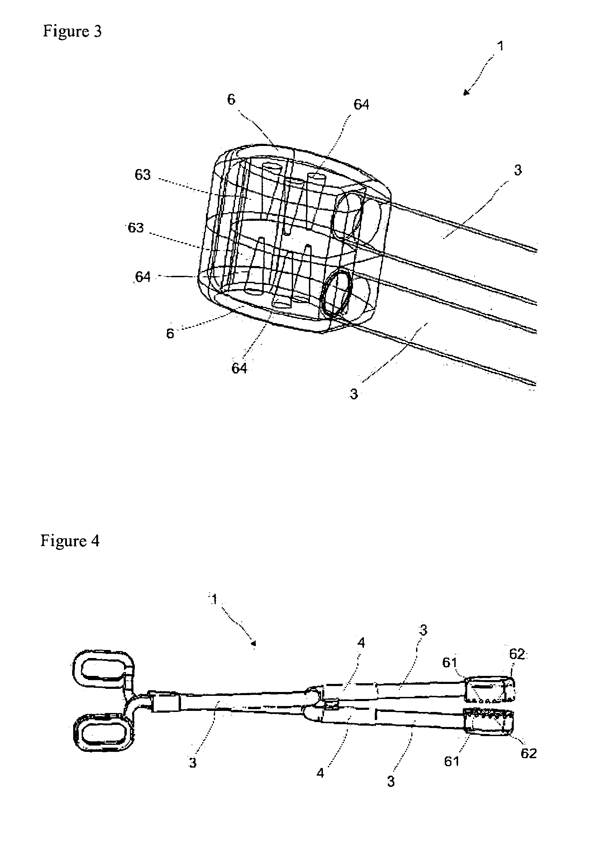

Figure 3 is the perspective view of the reservoir and the crusher.
Figure 4 is the side view of the forceps wherein the recesses and protrusions on the capsule are seen from the side.

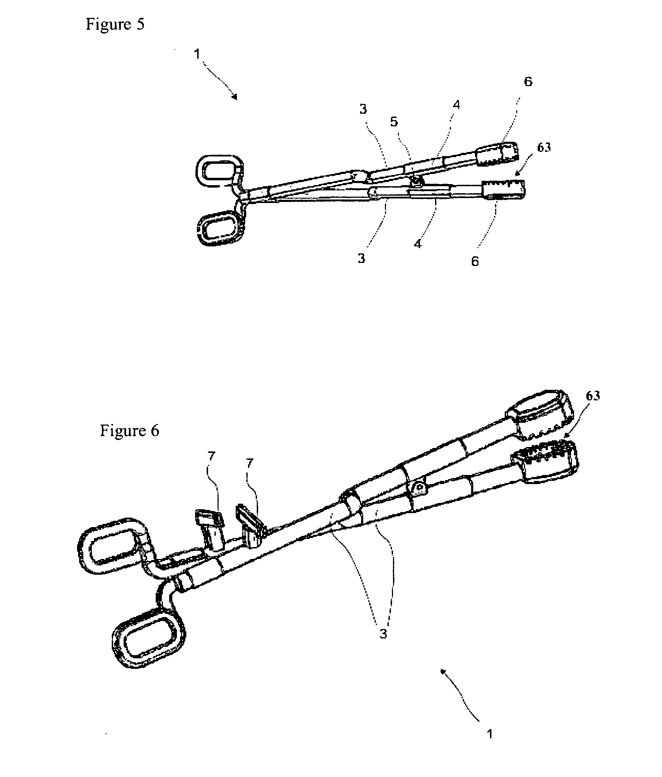

Figure 5 is the perspective view of the forceps at position wherein the connector is positioned close to the jaw and the jaw is in open position.
Figure 6 is the perspective view of the embodiment of the forceps that includes tap.

The components in the figures are each given a reference number and the numbers refer to the following.
1. Forceps
2. grip
   21. Connection member
   22. Ring
3. Tube
4. Connector
5. Hinge
6. Jaw
   61. Recess
   62. Protrusion
   63- Cavity
   64. Crusher
7. Valve

The inventive forceps (1) comprise:
- at least two jaws (6) which enable the stone to be grabbed and crushed in laparoscopic operations,
- at least two tubes (3) one end of one of which is fixed to each jaw (6), which enable the stones crushed inside the jaw (6) to be aspired,
- at least two connectors (4) which can move parallel to the axis of the tube (3) on each tube (3),
- at least one hinge (5) which is located between the connectors (4) and enables the angle between the tubes (3) to be changed,
- at least one grip (2) which is fixed to the end of the tube (3) that is not connected to the jaw (6), and which enables the user to move the tubes (3).

The grip (2) has at least two loops which enable it to apply force by holding it, at least two connection members (21) which enable the loops to be connected to the tubes (3) and at least two rings (22) which enable the connection member (21) to be fixed to the tube (3).

The user uses the forceps (1) by passing his/her fingers through the holder (2). The loops of the holder (2) are big enough for the user to pass his/her fingers through easily. The connection member (21) is preferably L shaped, and one end is fixed to the loop and the other end is fixed to the ring (22). The ring (22) is in the shape of a hollow cylinder and is fixed by being engaged onto the tube (3).

The jaws (6) are attached to the tubes (3) and the distance between the jaws (6) changes with the movement of the tube (3). One surface of each jaw (6) contacts the other. On the said surface there are plurality of recesses (61) and protrusions (62) which enable the jaws (6) to grip each other and the stones better. On the surface wherein the recesses (61) and the protrusions (62) are present there is a cavity (63) in which the crushed stones will spill. On the surface of the cavity (63) which is parallel to the surface wherein the recesses (61) and protrusions (62) are present, there is at least one crusher (64) extending from one jaw (6) to another. In the preferred embodiment of the invention, the crusher (64) is in the form of a cone. By means of the crusher (64) having a sharp structure, the stones stuck between the jaws (6) can be crushed via the crusher (64).

One end of tube (3) is connected to the ring (22) and the other end is connected to the jaw (6). The tube (3) is preferably in the form of "Z" which has right angle between its axis. The stones crushed in the jaw (6) pass through the tube (3) and are aspired inside the tube (3) via a hose which is connected to the tube (3) inside the ring (22).

In preferred embodiment of the invention, both ends of the tube (3) are open, there is at least one valve (7) present on the tube (3), which enables the user to control the flow therein. One end of the tube (3) faces the cavity (63). A hose can be connected to the end of the tube (3) which does not face the cavity (63), the flow between the tube (3) and the hose can be controlled by the valve (7). In case the hose is connected to any aspiration device, the stones gathered in the cavity (63) reach the hose by passing through the tube (3). Thus the forceps (1) are not required to be removed from the gallbladder for the stones to be extracted.

The connector (4) is a hollow cylinder, and it is placed such that it will be concentric with the tube (3). The connector (4) can move back and forth on the tube (3). There is at least one hinge (5) present between the connectors (4). The hinge (5) keeps the connectors (4) and the tubes (3), to which the connectors (4) are connected, together. The tubes (3) moves like a scissor by rotating on the axis of the hinge (5). During this movement, the jaws (6) approach to each other and diverge.

By means of the rotation of the connector (4) on tube's (3) axis, the distance between the hinge (5) and the jaws (6) changes. Thus the position of the connector (4) changes the distance between the jaws (6) and the range between the jaws (6). When the connector (4) approaches the jaw (6), the jaws (6) can move less; whereas when the connector (4) approaches the holder (2), the distance between the jaws (6) can be more. Also when the connector (3) is close to the jaw (6), low force applied by the user to the holder (2) generates higher force on the jaw (6). Thus the user can easily crush the stones.

In laparoscopic surgery first the forceps (1) are introduced into the gallbladder. The user passes his/her fingers through the holder (2), moves the circles apart, by this means enables the jaws (6) to be opened with motion of the tubes (3). After the jaws (6) move apart, the stone is pinched between two jaws (6). While the jaws (6) are moved apart from each other, the hinge (5) and the connector (4) being close to the holder (2) enables the larger stones to be grabbed. When the user approaches the circles to each other, the holder (64) applies pressure to the stone and when it reaches the sufficient force, it crushes the stone. While the jaws (6) are moved close to each other, the hinge (5) and the connector (4) being close to the holder (6) enables the larger stones to be crushed by applying less force. The crushed stone spills into the cavity (63). The stones filling the cavity (63) pass inside the tube (3) from the cavity (63), and are extracted out from the tube (3) via the hoses. If there is obstruction during the surgery, the cavity (63) and the tubes (3) can be cleaned by being washed.

There is no need to introduce the forceps (1) into the gallbladder many times, because the stones minimized by being crushed inside the jaw (6) are aspirated and taken inside the tube (3). By this means a risk such as puncturing the gallbladder is minimized.

It is possible to develop various embodiments of the inventive forceps (1). The invention can not be limited to the examples described herein and it is essentially according to the claims.

## Claims

1. Forceps (1) used in laparoscopic gallstone surgeries; comprising
- at least two jaws (6) which enable the stone to be grabbed and crushed;
- at least two tubes (3) one end of one of which is fixed to each jaw (6), which enable the jaws (6) to move by transferring the movement of the user to the jaws (6);
- at least one hinge (5) which enables the angle between the tubes (3) to be changed;
- at least two grip (2) which are fixed to the ends of respective tubes (3) not connected to a jaw (6), and which enable the user to move the tubes (3) about the hinge (5); and **characterized by**
- at least two sliding connectors (4) which can move parallel to the axis of the tube (3) on respective tubes (3) and which are attached to respective parts of the hinge (5);
- the proximal end of each tube (3) being adapted for connection to a vacuum source which enables the crushed stones in the jaws (6) to be aspired therethrough.

2. Forceps (1) according to claim 1, **characterized by** holder (2) which has at least two circles which enable it to apply force by holding it, at least two connection members (21) which enable the circles to be connected to the tubes (3) and at least two rings (22) which enable the connection member (21) to be fixed to the tube (3).

3. Forceps (1) according to any one of the claims 1 or 2, **characterized in that** jaws (6) comprise a plurality of recesses (61) and protrusions (62) on surfaces contacting each other, enabling each other and stones to be gripped better.

4. Forceps (1) according to claim 3, **characterized in that** jaws (6) comprise at least one cavity (63) in which the crushed stones will spill, on the surface wherein the recesses (61) and the protrusions (62) are present.

5. Forceps (1) according to claim 4, **characterized in that** jaws (6) comprise at least one crusher (64) inside the cavity (63) extending from a surface parallel to the surface wherein the recesses (61) and the protrusions (62) are present towards the surface wherein the recesses (61) and the protrusions (62) are present.

6. Forceps (1) according to any one of the preceding claims, **characterized in that** tube (3) is a Z shaped tube (3) one end of which is connected to a ring (22) and which has right angles between the axes of its legs.

7. Forceps (1) according to any one of the preceding claims, **characterized in that** tube (3) has at least one value (7) thereon enabling the flow inside to be controlled by the user.

8. Forceps (1) according to any one of the preceding claims, **characterized in that** each connector (4) is in the form of a hollow cylinder mounted concentric with a tube (3) and which can move back and forth on the tube (3).

## Patentansprüche

1. Zange (1), die in der laparoskopischen Gallenstein-Chirurgie verwendet wird, umfasst
- mindestens zwei Backen (6), welche ermöglichen, die Steine zu greifen und zu brechen;
- mindestens (3) zwei Röhre, ein Ende von einer von derer jeweils an einer Backen (6) befestigt ist, welche es ermöglichen, dass die Backen (6) sich bewegen, indem die Bewegung des Benutzers auf diese Backen übertragen wird (6);
- mindestens ein Gelenk (5), welches ermöglicht den Winkel zwischen den Röhren (3) zu ändern;
- mindestens zwei Griffe (2), die an den Enden des jeweiligen Rohres (3) befestigt sind, welches mit einem Backen (6) nicht verbunden ist und welches dem Benutzer die Möglichkeit bietet, die Röhre (3) um das Gelenk (5) zu bewegen und **gekennzeichnet durch**
- mindestens zwei gleitende Verbindungsglieder (4), die zur Achse des Rohres (3) parallel auf dem jeweiligen Rohr (3) bewegt werden können und an den jeweiligen Teilen des Gelenks (5) befestigt sind;
- das proximale Ende des jeden Rohres (3) ist zur Verbindung mit einer Saugluftquelle angepasst, welche ermöglicht, die gebrochenen Steine in den Backen (6) anzusaugen.

2. Zange (1) nach Anspruch 1, **gekennzeichnet durch** Halterung (2), die zumindest zwei Kreise aufweist, die ermöglichen, dass dieser Kraft ausübt, wenn sie festgehalten wird; zumindest zwei Verbindungsglieder (21), die es erlauben, dass die Kreise an den Röhren (3) angeschlossen werden und zumindest zwei Ringe (22), die es ermöglichen, dass die Verbindungsglieder (21) an dem Rohr (3) angeschlossen werden.

3. Zange (1) nach einem der Ansprüche 1 oder 2, **dadurch gekennzeichnet, dass** die Backen eine Vielzahl von Aussparungen (61) und Vorsprünge (62) auf den miteinander kontaktierenden Oberflächen aufweisen, die es erlauben, dass diese einander selbst sowie die Steine besser greifen.

4. Zange (1) nach Anspruch 3, **dadurch gekennzeichnet, dass** Backen (6) auf der Oberfläche, wo die Aussparungen (61) und die Vorsprünge (62) vorhanden sind, mindestens eine Aushöhlung (63) aufweisen, in die die zerbrochenen Steine verschüttet werden.

5. Zange (1) nach Anspruch 4, **dadurch gekennzeichnet, dass** die Backen (6) mindestens einen Brecher (64) innerhalb der Aushöhlung aufweisen, der sich von einer Oberfläche, die zur Oberfläche parallel verläuft, worin die Aussparungen (61) und Vorsprünge (62) vorhanden sind, bis zur Oberfläche erstreckt, worin die Aussparung sowie die Vorsprünge (62) liegen.

6. Zange (1) nach einem der vorhergehenden Ansprüche, **dadurch gekennzeichnet, dass** das Rohr (3) ein Z-förmiges Rohr darstellt, dessen ein Ende an einem Ring (22) angeschlossen ist und welches rechte Winkel zwischen seinen Beinen aufweist.

7. Zange (1) nach einem der vorhergehenden Ansprüche, **dadurch gekennzeichnet, dass** das Rohr (3) mindestens einen Hahn (7) darauf aufweist, welcher dem Benutzer erlaubt den Fluss im Innern zu kontrollieren.

8. Zange (1) nach einem der vorhergehenden Ansprüche, **dadurch gekennzeichnet, dass** jedes Verbindungsglied (4), das in Form eines hohlen Zylinders ist, konzentrisch mit einem Rohr (3) angebaut wird und auf dem Rohr (3) hin und her bewegt werden kann.

## Revendications

1. Forceps (1) utilisé dans la chirurgie de calcul biliaire laparoscopique ; comportant
- au moins deux mâchoires (6) permettant au rocher d'être saisi et écrasé ;
- au moins deux tuyaux (3) dont l'une de ses extrémités étant fixée à chaque mâchoire (6), permettant aux mâchoires (6) à se déplacer en transférant le mouvement de l'utilisateur aux mâchoires (6) ;
- au moins une charnière (5) permettant l'angle entre les tuyaux (3) à changer ;
- au moins deux poignées (2) fixées aux extrémités de tuyaux respectifs (3) n'étant pas liées à une mâchoire (6), et permettant à l'utilisateur de remuer les tuyaux (3) autour de la charnière (5) ; et **caractérisé par**
- au moins deux connecteurs coulissants (4) pouvant se déplacer parallèlement à l'axe du tuyau (3) sur les tuyaux respectifs (3) et étant fixés aux parties respectives de la charnière (5) ;
- l'extrémité proximale de chaque tuyau (3) permettant aux rochers écrasés dans les mâchoires (6) d'être aspirés.

2. Forceps (1) selon la revendication 1, **caractérisé par** la poignée (2) possédant au moins deux cercles permettant l'application de la force en la détenant, au moins deux membres de connexion (21) permettant aux cercles d'être liés aux tuyaux (3) et au moins deux bagues (22) permettant la fixation du membre de connexion (21) au tuyau (3).

3. Forceps (1) selon l'une quelconque des revendications 1 ou 2, **caractérisé par le fait que** les mâchoires (6) comportent une pluralité de renfoncements (61) et protrusions (62) sur les surfaces étant en contact avec l'une et l'autre, permettant l'une et l'autre et les rochers d'être saisi mieux.

4. Forceps (1) selon la revendication 3, **caractérisé par le fait que** les mâchoires (6) comportent au moins une cavité (63) dans laquelle les rochers écrasés seraient répandus, sur la surface dans laquelle les renfoncements (61) et protrusions (62) sont présents.

5. Forceps (1) selon la revendication 4, **caractérisé par le fait que** les mâchoires (6) comportent au moins un broyeur (64) à l'intérieur de la cavité (63) s'étendant d'une surface parallèle à la surface dans laquelle sont présents les renforcements (61) et les protrusions (62) vers la surface dans laquelle sont présents les renforcements (61) et protrusions (62).

6. Forceps (1) selon l'une quelconque des revendications précédentes, **caractérisé par le fait que** le tuyau est un tuyau (3) en forme de Z dont l'une de ses extrémités étant liée à une bague (22) et possédant des angles corrects entre les axes de ses pieds.

7. Forceps (1) selon l'une quelconque des revendications précédentes, **caractérisé par le fait que** le tuyau (3) possède au moins une valve (7) permettant le control du flux intérieur par l'utilisateur.

8. Forceps (1) selon l'une quelconque des revendications précédentes, **caractérisé par le fait que** chaque connecteur (4) est en forme d'un cylindre creux monté de façon concentrique avec une tuyau (3) et pouvant reculer et avancer sur le tuyau (3).
